# EUROPEAN PATENT APPLICATION

(11) **EP 4 785 850 A1**
(43) Date of publication of application: **05.08.2026**
(21) Application number: 24872152.4
(22) Date of filing: 24.09.2024
(51) Int. Cl.: A61B 1/018, A61B 1/01, G02B 23/24

(54) **ENDOSCOPIC TREATMENT TOOL AND ENDOSCOPE SYSTEM**

(30) Priority: 29.09.2023 JP 2023169022
(71) Applicant: Nipro Corporation, Settsu-shi, Osaka 566-8510 (JP)
(72) Inventor: MATSUMOTO Misa, Settsu-shi, Osaka 566-8510 (JP); MIYAGAWA Katsuya, Settsu-shi, Osaka 566-8510 (JP)
(74) Representative: Wunderlich & Heim Patentanwälte PartG mbB
(86) International application number: PCT/JP2024/033865
(87) International publication number: WO 2025/070361

(57) **Abstract**

An endoscopic treatment instrument (20) comprises a catheter shaft (21) having a first channel (23) into which an endoscope (10) is inserted and a second channel (24) for use in a prescribed treatment, and a handle (22) attached to the base end of the catheter shaft (21). At the leading end of the catheter shaft (21), a leading end marker (32) for confirming the top-bottom direction of the endoscope (10) is provided to a portion of the inner circumferential surface of the first channel (23).

## Description

### TECHNICAL FIELD

The present invention relates to an endoscopic treatment instrument and endoscope system.

### BACKGROUND

Conventionally, endoscope systems for performing a predetermined treatment while observing the inside of the body have been known. For example, Patent Document 1 discloses an endoscopic treatment instrument provided with a guide pipe into which an endoscope is inserted and a guide sheath mounted to a distal end portion of the pipe, and an endoscope system using the treatment instrument. Patent Document 1 describes that forceps, a brush, or the like can be passed through the guide sheath to collect mucosal tissue from the paranasal sinuses.

### CITATION LIST

### PATENT LITERATURE

Patent Document 1: JP 6082169 B

### SUMMARY

It is important that endoscopic treatment instruments and endoscope systems be easy to operate and use. Although the endoscope system according to Patent Document 1 has a relatively simple structure and improved operability, there is a need to further improve the usability to reduce the burden on a doctor using the instrument.

An endoscopic treatment instrument according to an aspect of the present invention includes a catheter shaft that has a first channel into which an endoscope is inserted and a second channel used for a predetermined treatment, and a handle that is attached to a proximal end portion of the catheter shaft, and in this instrument, at a distal end portion of the catheter shaft, a distal end marker for checking the top-and-bottom direction of the endoscope is provided on a portion of an inner circumferential surface of the first channel.

According to the above configuration, it is possible to easily confirm the top-and-bottom direction of the endoscope inserted in the first channel of the catheter shaft by means of the distal end marker. Once the catheter shaft is inserted into the body, it may become difficult to determine the top and bottom of the endoscope. In this case, providing the distal end marker makes it possible to confirm the top and bottom of the endoscope at the distal end portion of the catheter shaft. In other words, with the above configuration, even when it becomes difficult to determine the top and bottom of the endoscope, the usability of the treatment instrument is improved significantly as there is no need to pull the endoscope out of the first channel, confirm its top and bottom, and then reinsert it. In addition, even during initial setup when the endoscope is inserted into the first channel, the top-and-bottom direction of the endoscope can be checked by means of the distal end marker.

An endoscopic treatment instrument according to another aspect of the present invention includes a catheter shaft that has a first channel into which an endoscope is inserted and a second channel used for a predetermined treatment, a handle that is attached to a proximal end portion of the catheter shaft, and a first tube that constitutes the first channel and extends from a proximal end side of the handle, and in this instrument the first tube is provided with a locking portion that locks relative movement of the endoscope with respect to the catheter shaft.

With the above configuration, it is possible to prevent the endoscope from rotating within the first channel while the endoscope system is in use. In other words, the locking portion can function to maintain the endoscope inserted and set in the first channel in a more reliable manner. When the endoscope rotates within the first channel with the catheter shaft inserted in the body, it may become difficult to determine the top-and-bottom direction of the endoscope. The above configuration solves this problem and significantly improves the usability of the treatment instrument.

An endoscopic treatment instrument according to another aspect of the present invention includes a catheter shaft that has a first channel into which an endoscope is inserted and a second channel used for a predetermined treatment, a handle that is attached to a proximal end portion of the catheter shaft, and a first tube that constitutes the first channel and extends from a proximal end side of the handle, and in this instrument the first tube is provided with a branch port for irrigation to supply a cleaning solution to a distal end of the endoscope through the first channel of the catheter shaft.

With the above configuration, it is possible to supply a cleaning solution to the first channel in which the endoscope is inserted, and thereby effectively clean the distal end of the endoscope. Although the distal end of the endoscope may be contaminated due to adhesion of bodily fluids or the like, and this makes it difficult to acquire clear images, the above configuration allows the cleaning solution to be supplied to the distal end of the endoscope more reliably and efficiently than, for example, a case wherein the cleaning solution is supplied through a separate channel. As such, irrigation of the distal end of the endoscope becomes easier, and the usability of the treatment instrument is improved significantly.

The handle is preferably provided with a rotational operating portion for bending the distal end portion of the catheter shaft. The handle is also preferably provided with a hand position marker.

With the above configuration, it is possible to bend the distal end portion of the catheter shaft by means of the rotational operating portion of the handle. Thus, it becomes possible to improve the operability of the treatment instrument and facilitate observation and treatment of a target site inside the body. The hand position marker of the handle can serve as, for example, an indicator of a position of the rotational operating portion and can also be used to check the top-and-bottom direction of the endoscope.

An endoscope system according to one aspect of the present invention is characterized by including the above endoscopic treatment instrument and the endoscope inserted into the first channel.

The endoscopic treatment instrument and the endoscope system according to the present invention are simple to operate and easy to use, and can thus reduce the burden on physician users.

### BRIEF DESCRIPTION OF DRAWINGS

FIG. 1 is a diagram showing the overall configuration of an endoscope system according to an example of embodiments;
FIG. 2 is a perspective view of an endoscopic treatment instrument according to an example of the embodiments;
FIG. 3 is a diagram showing a distal end surface of a catheter shaft of the endoscopic treatment instrument;
FIG. 4 is a diagram showing a proximal end side portion of the endoscopic treatment instrument;
FIG. 5 is an exploded perspective view of a handle of the endoscopic treatment instrument;
FIG. 6 is a diagram showing an endoscope according to an example of the embodiments;
FIG. 7 is a cross-sectional view taken along the longitudinal direction of a portion of the endoscope having a protective tube;
FIG. 8 is a cross-sectional view taken along line AA in FIG. 7; and
FIG. 9 is a diagram showing an example of use of the endoscope system according to an example of the embodiments.

### DESCRIPTION OF EMBODIMENTS

Embodiments of an endoscopic treatment instrument and endoscope system according to the present invention will be described in detail below with reference to the drawings. The embodiments described below are merely examples, and the present invention is not limited to these embodiments. In addition, the present invention includes any form obtained by selectively combining the embodiments and modifications described below.

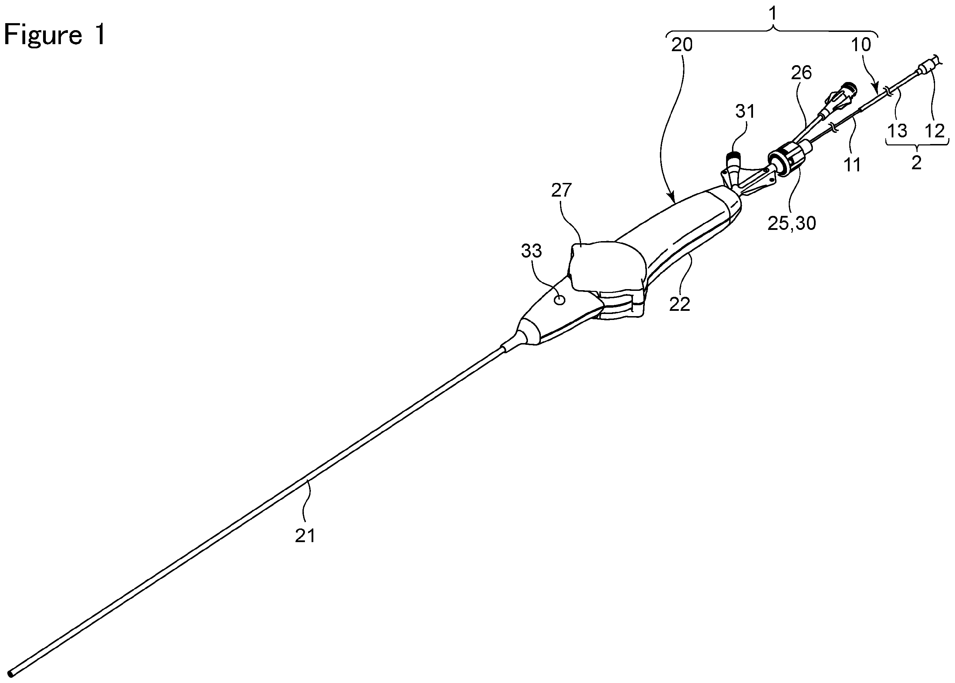

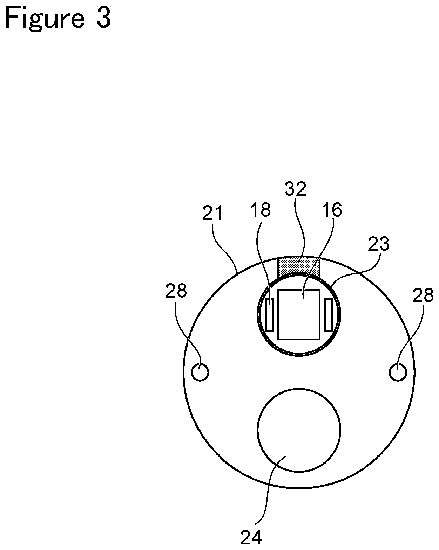

FIG. 1 is a perspective view showing the overall configuration of an endoscope system 1 according to an example of embodiments. As shown in FIG. 1, the endoscope system 1 includes an endoscope 10 and an endoscopic treatment instrument 20 and is configured such that the endoscope 10 can be inserted into a first tube 25 of the endoscopic treatment instrument 20. The endoscope 10 is a flexible endoscope including a flexible and bendable insertion portion 11. The first tube 25 extends to a distal end of a catheter shaft 21 and forms a first channel 23 (see FIG. 3 below) through which the insertion portion 11 of the endoscope 10 passes. The endoscope system 1 is typically used with a distal end of the insertion portion 11 inserted up to a distal end of the catheter shaft 21 of the endoscopic treatment instrument 20.

The endoscope system 1 can be employed in various methods of performing endoscopic treatments, such as arthroscopy, laryngoscopy, transoral endoscopy, transnasal endoscopy, cholangioscopy, colonoscopy, and body cavity endoscopy. In the present embodiment, the use of the endoscope system 1 in the Racz catheter method will be specifically described as an example. The Racz catheter method is a treatment for chronic low back pain due to spinal canal stenosis, herniated discs, or the like. It involves inserting a catheter close to a painful site to remove adhesions around the nerves. The usage of the endoscope system 1 will be described in detail below.

The endoscope system 1 includes a console that controls the endoscope 10 and a monitor that displays images acquired by the endoscope 10 (both not shown). The console receives electrical signals from, for example, an imaging unit 16 of the endoscope 10 (see FIG. 3 below) and generates images. The generated images are displayed on the monitor, and the physician can thus perform a predetermined treatment while observing the inside of the body. The console also includes an illumination light source. The console and the monitor may be integrated. The illumination device is not limited to the one built in the console and may be a separate device.

The endoscope 10 constituting the endoscope system 1 includes the insertion portion 11 and a base portion 2. The insertion portion 11 is a portion to be inserted into the body. The insertion portion 11 is inserted into the catheter shaft 21 of the endoscopic treatment instrument 20, and then into the body. Typically, the endoscope 10 is inserted into the endoscopic treatment instrument 20 such that the distal end of the insertion portion 11 and the distal end of the catheter shaft 21 are substantially aligned. The base portion 2 is a portion disposed adjacent to the proximal end side of the insertion portion 11 and has a connector portion 12 and a protective tube 13.

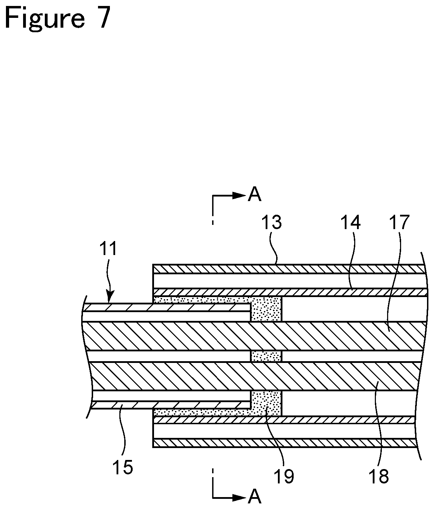

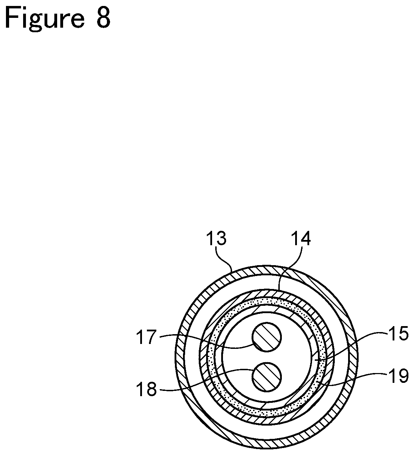

The connector portion 12 of the endoscope 10 is a portion to which a cable extending to the console is connected. The connector portion 12 is also connected to a cable extending from a proximal end portion of the insertion portion 11. Although, in the insertion portion 11 inserted in the endoscopic treatment instrument 20, the cable is contained in an outer tube 15 (see FIGs. 7 and 8 below), a portion of the cable extends out of the proximal end of the outer tube 15 and connects to the connector portion 12. The endoscope 10 includes a protective tube 13 that covers the cable exposed from the outer tube 15.

The endoscopic treatment instrument 20 constituting the endoscope system 1 includes the catheter shaft 21 and a handle 22. As described in detail below, the catheter shaft 21 is an elongated tube having a first channel 23 into which the endoscope 10 is inserted and a second channel 24 used for a predetermined treatment. The second channel 24 allows a medicinal solution to flow therethrough and allows instruments such as forceps or a brush to be inserted therein. The second channel 24 also makes it possible to suck body fluids therethrough. At least a distal end section of the catheter shaft 21 is made of a flexible material that can be bent.

The handle 22 of the endoscopic treatment instrument 20 is a portion to be grasped during use and is attached to a proximal end portion of the catheter shaft 21. The handle 22 has a shape that is thicker and easier to grip than the catheter shaft 21 and is, for example, made of a rigid resin material. The handle 22 is provided with a rotational operating portion 27 for bending a distal end portion of the catheter shaft 21. The handle 22 is also provided with a hand position marker 33. As described in detail below, the hand position marker 33 serves as an indicator indicating, for example, a position of the rotational operating portion 27.

The endoscopic treatment instrument 20 further includes two tubes, a first tube 25 and a second tube 26, extending from the proximal end side of the handle 22. The first tube 25 constitutes the first channel 23 into which the endoscope 10 is inserted. The second tube 26 constitutes the second channel 24 into which instruments such as forceps or a brush are inserted. The two tubes are integrated within the handle 22 to thereby form the single catheter shaft 21. The first tube 25 is provided with a locking portion 30 that locks the endoscope 10 and a branch port 31 for irrigation. The second tube 26 is provided with a connector 29 at its proximal end.

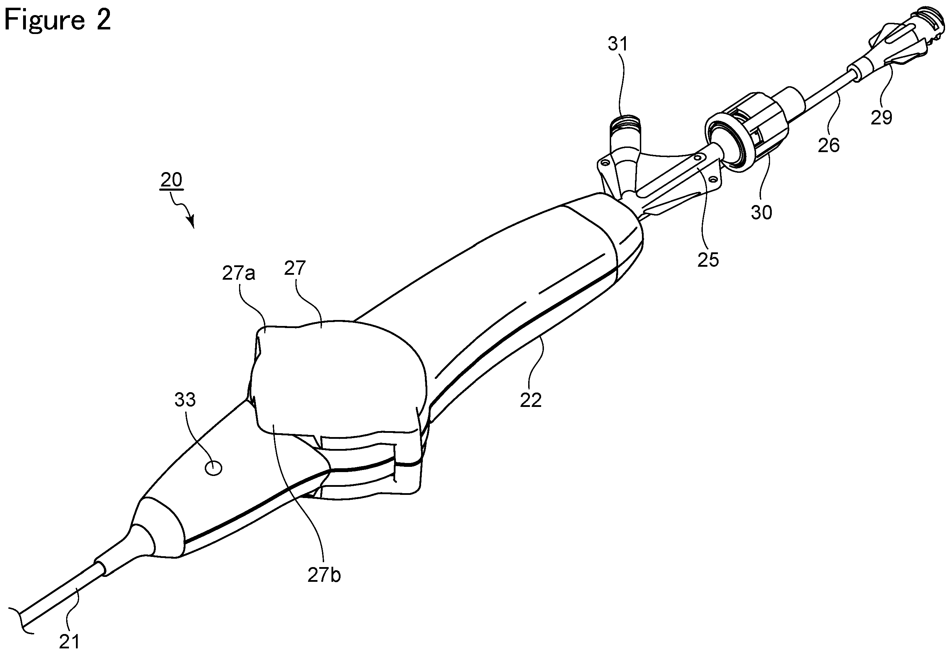

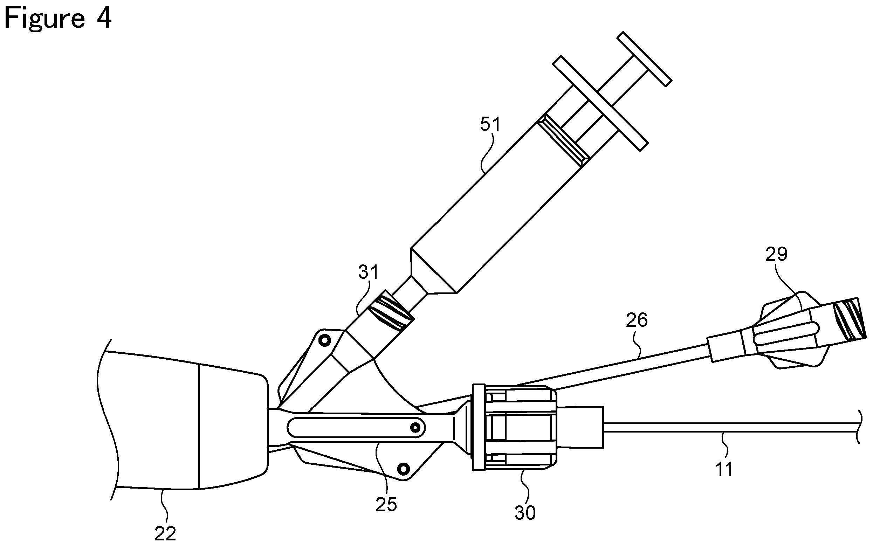

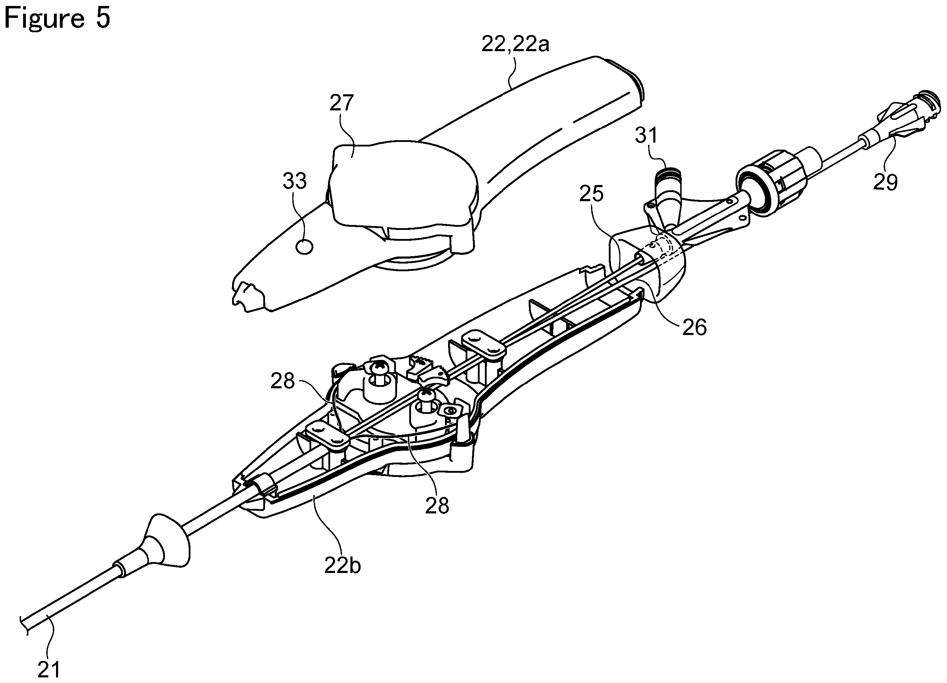

The configuration of the endoscope system 1, in particular the configuration of the endoscopic treatment instrument 20, will be described in detail below with reference to FIGs. 2 to 5. FIG. 2 is a perspective view showing the endoscopic treatment instrument 20. FIG. 3 is a diagram showing a distal end surface of the catheter shaft 21. FIG. 4 is an enlarged view of a proximal end side portion of the endoscopic treatment instrument 20. FIG. 5 is an exploded perspective view of a portion of the handle 22.

As shown in FIGs. 2 to 5, the endoscopic treatment instrument 20 has a structure in which the catheter shaft 21 extends from a distal end of the handle 22, and the two tubes extend from a proximal end of the handle 22. The catheter shaft 21 is preferably longer than the handle 22. When the endoscopic treatment instrument 20 is applied in the Racz catheter method, a suitable length of the catheter shaft 21 is, for example, 300 mm to 400 mm. The length of the handle 22 is, for example, 60% or less of the length of the catheter shaft 21. The first tube 25 and the second tube 26 extend from the proximal end of the handle 22 by a length shorter than the length of the handle 22.

A distal end portion of the insertion portion 11 of the endoscope 10 to be inserted into the catheter shaft 21 has a built-in imaging unit 16 including an imaging device. The imaging unit 16 may include a lens, filter, cover glass, or the like. The imaging unit 16 has, for example, a generally square light-receiving surface and is provided such that the light-receiving surface is located on a distal end surface of the insertion portion 11. The imaging device is a device in which a plurality of photodiodes converting light into electrical signals are arranged on a substrate. The imaging device is also referred to as a solid-state imaging device or image sensor.

Suitable examples of the imaging device include a CCD image sensor and a CMOS image sensor. Light incident on the imaging device is converted into electrical signals, and acquired image data is transmitted to the console. The distal end surface of the insertion portion 11 further has an optical fiber 18. The optical fiber 18 is a light guide for directing light from the illumination light source to the distal end surface of the insertion portion 11. For the endoscope 10, light emitted from the optical fiber 18 is irradiated onto and reflected from a target site to be observed. The reflected light then enters the imaging device, and image data of the target site is thus acquired.

The catheter shaft 21 is a thin tube having a substantially constant outer diameter over its entire length. The catheter shaft 21 is to be inserted into the body, and therefore, it is preferable that it has a small outer diameter. The outer diameter of the catheter shaft 21 is, for example, 2.0 mm to 3.5 mm, or 2.3 mm to 3.0 mm.

The catheter shaft 21 is made of a flexible material as described above. The material of the catheter shaft 21 is not particularly limited, but a suitable example includes a polyamide-based thermoplastic elastomer with good biocompatibility (e.g., polyamide polyamine epichlorohydrin (PAE)). The catheter shaft 21 may contain an X-ray contrast agent, such as barium sulfate. The catheter shaft 21 may also contain various pigments to be colored.

The hardness of the distal end of the catheter shaft 21 may differ from its hardness in other sections. In the present embodiment, the hardness of the distal end section of the catheter shaft 21 is lower than that of the other sections, and the distal end section is configured to be bendable by operating the rotational operating portion 27. The hardness of the distal end section may be 60% or less of that of the other sections. The length of the distal end section, which has a lower hardness than the other sections, is, for example, 5% to 20% of the total length of the catheter shaft 21. Making the catheter shaft 21 bendable over a predetermined length range from its distal end facilitates observation and treatment of the target site inside the body.

As described above, the catheter shaft 21 has the first channel 23 into which the endoscope 10 is inserted and the second channel 24 used for a predetermined treatment (see FIG. 3). The first channel 23 has a diameter that allows the endoscope 10 to be inserted therethrough, and is formed over the entire length of the catheter shaft 21. The second channel 24 has a diameter that allows instruments such as forceps or a brush to be inserted therein and is formed parallel to the first channel 23 over the entire length of the catheter shaft 21. Although the catheter shaft 21 may have three or more channels, in the present embodiment, it has a double-lumen structure containing the two channels. The diameters of the two channels may be the same and, for example, from 1.0 mm to 1.5 mm.

In the catheter shaft 21, two wires 28 are introduced to the distal end of the catheter shaft 21 to bend its distal end section by operating the rotational operating portion 27 (see FIGs. 3 and 5). The catheter shaft 21 is manufactured by, for example, extrusion molding a polyamide-based thermoplastic elastomer so as to integrate the two tubes constituting the two channels with the two wires 28. These two tubes are the first tube 25 and the second tube 26 extending from the proximal end of the handle 22. The proximal end of the catheter shaft 21 is located within the handle 22. That is, the two tubes and the two wires 28 are integrated within the handle 22.

At the distal end portion of the catheter shaft 21, a distal end marker 32 for checking the top-and-bottom direction of the endoscope 10 is provided on a portion of an inner circumferential surface of the first channel 23, into which the endoscope 10 is inserted (see FIG. 3). Although the endoscope 10 is inserted into the first channel 23 with its top-and-bottom direction specified and is set into the endoscopic treatment instrument 20 such that the distal ends of the insertion portion 11 and the catheter shaft 21 are aligned, it may become difficult to determine the top and bottom of the endoscope 10 during use. In such situations, provision of the distal end marker 32 makes it possible to easily confirm the top and bottom of the endoscope 10. Usage of the distal end marker 32 will be described below.

The distal end marker 32 is preferably provided on the inner circumferential surface of the first channel 23 over a range of half or less of the length of the inner circumference, and may have a size that can be seen in images acquired by the endoscope 10. The distal end marker 32 is, for example, an indicator indicating the upper side (top) of the endoscope 10 and is provided on the upper half of the inner circumferential surface of the first channel 23. Although the distal end marker 32 may be provided on the inner circumferential surface of the first channel 23, in the present embodiment, the distal end marker 32 is provided so as to extend from the inner circumferential surface of the first channel 23 to an outer circumferential surface of the catheter shaft 21 which is adjacent to the inner circumferential surface. In this case, a position of the distal end marker 32 can be easily checked from the outside of the catheter shaft 21.

Although the distal end marker 32 may be provided near the distal end of the catheter shaft 21 at a position spaced apart from the distal end, it is preferably provided over a predetermined length range from the distal end of the catheter shaft 21. The length of the distal end marker 32 along the longitudinal direction of the catheter shaft 21, i.e., the above predetermined length, is not particularly limited. However, the length of the distal end marker 32 is preferably set to 0.5 mm or more because the distal end marker 32 becomes difficult to be checked if made too small. The length of the distal end marker 32 is preferably 1 mm or more, and for example, 1 mm to 5 mm, or 2 mm to 4 mm. The distal end marker 32 is provided, for example, only on the distal end section of the catheter shaft 21, which has a reduced hardness.

The distal end marker 32 can be provided by adding a predetermined coloring material to a resin of which the catheter shaft 21 is composed. The predetermined coloring material may be any material that has a color different from the pigment coloring the entire catheter shaft 21 or other additives, such as barium sulfate. The predetermined coloring material may be a general pigment or resin particles, inorganic particles, metal particles, or the like that are not generally used as pigments. When a metal, such as a platinum-iridium alloy, is used as the coloring material, the position of the distal end marker 32 can be easily detected under X-ray fluoroscopy. However, when metal is used, the catheter shaft 21 tends to become stiffer, and it is then preferable to make the size of the distal end marker 32 smaller.

The distal end marker 32 is disposed in the same direction as the top surface of the handle 22 (described below) with respect to the endoscope 10 inserted in the first channel 23. In the present embodiment, the endoscopic treatment instrument 20 is used with the top surface of the handle 22 facing vertically upward, and, at this time, the distal end marker 32 indicating the top of the endoscope 10 also faces vertically upward. As long as the insertion portion 11 of the endoscope 10 does not rotate within the first channel 23, the top and bottom of the endoscope 10 can then be determined from the orientation of the top surface of the handle 22.

The distal end marker 32 is checked in images acquired by the endoscope 10. During the initial setup when the endoscope 10 is inserted into the first channel 23, the top-and-bottom direction of the endoscope 10 can be checked by means of the distal end marker 32. The endoscope 10 is set into the endoscopic treatment instrument 20 such that the distal end of the insertion portion 11 is aligned with the distal end of the catheter shaft 21, and therefore, when the top and bottom of the endoscope 10 becomes unclear, the endoscope 10 is moved to the proximal end side by a few millimeters to thereby capture an image of the distal end marker 32.

In the endoscopic treatment instrument 20, the distal end marker 32 allows the top and bottom of the endoscope 10 to be checked simply by slightly moving the insertion portion 11 of the endoscope 10. For example, when the distal end marker 32 appears on the left side of an image displayed on the monitor, it indicates that the top of the endoscope 10 is tilted to the left side. In this case, the top-and-bottom direction of the endoscope 10 can be readjusted by rotating the endoscope 10 such that the distal end marker 32 appears on the upper side on the monitor screen. In other words, the distal end marker 32 is an indicator for checking the top and bottom of the image acquired by the endoscope 10. If the distal end marker 32 were absent, it would be necessary to completely pull the endoscope 10 out of the endoscopic treatment instrument 20, check its top and bottom, and then reinsert it.

The handle 22 has a thickness that makes it easy to grip, and is formed in a flat cylindrical shape that is long in the direction in which the catheter shaft 21 extends. A housing that forms the handle 22 has a rounded shape without corners for ease of grip, and the side surfaces of the housing are gently curved so as to project inward. In the present embodiment, the up and down directions of the handle 22 are specified. For example, the surface on which the manufacturer name, the manufacturer logo, the product name, and the like are displayed is the top surface of the handle 22, and the endoscopic treatment instrument 20 is used with the top surface of the handle 22 oriented vertically upward. As shown in FIG. 5, the housing of the handle 22 includes a top case 22a and a bottom case 22b and is configured to be disassembled.

The handle 22 has a maximum thickness (maximum length in the up-and-down direction) of, for example, 20 mm to 30 mm, and the thickness is largest at the portion where the rotational operating portion 27 is provided. The handle 22 is formed in a tapered shape that gradually narrows from the rotational operating portion 27 toward the distal end side and the proximal end side of the handle 22. The rotational operating portion 27 is disposed such that the center of the rotational operating portion 27 is located within a range of 70 mm to 90 mm from the distal end of the handle 22 and a range of 80 mm to 100 mm from the proximal end of the handle 22. The handle 22 is easy to hold and provides good operability.

As described above, the handle 22 has the rotational operating portion 27 on the distal end side relative to the center in the longitudinal direction. The rotational operating portion 27 is an operating portion that is generally disc-shaped overall, and parts of its outer circumferential edge are shaped to protrude in the left-and-right direction and the distal end direction of the handle 22. The physician can rotate the rotational operating portion 27 left or right, thereby bending the distal end portion of the catheter shaft 21 left or right. The rotational operating portion 27 has two wires 28 that are fixed thereto and extend to the distal end of the catheter shaft 21. When the rotational operating portion 27 is rotated left or right, one of the wires 28 is pulled to cause the distal end portion of the catheter shaft 21 to bend in the direction the rotational operating portion 27 is rotated.

A protruding portion 27a of the rotational operating portion 27 extending in the right-and-left direction from the handle 22 makes it easier for the rotational operating portion 27 to be caught by hand and improves operability. In addition, a protruding portion 27b extending in the direction of the distal end of handle 22 serves as an indicator indicating a position of the rotational operating portion 27. When the protruding portion 27b faces straight in the distal end direction, the rotational operating portion 27 is in its neutral position, and the distal end portion of the catheter shaft 21 is in a straight state. When the protruding portion 27b faces the left or right of the handle 22, the distal end portion of the catheter shaft 21 is bent in the direction in which the protruding portion 27b faces. The shapes of the rotational operating portion 27 when viewed from both the upper and lower sides of the handle 22 are the same, and the protrusions 27b are formed on the top and bottom of the handle 22.

It is preferable that the rotational operating portion 27 be configured to maintain the orientation of the operating portion (the orientation of the protruding portions 27b) even when the hand is released. With such a configuration, the distal end portion of the catheter shaft 21 can remain bent left or right even after the hand is released from the rotational operating portion 27, thereby improving operability. At the same time, it is also preferable that the torque of the rotational operating portion 27 is set such that the rotational operating portion 27 rotates smoothly without applying excessive force when the rotational operating portion 27 is rotated with a finger hooked on the protruding portion 27a.

The handle 22 has the hand position marker 33 on the distal end side relative to the rotational operating portion 27. The hand position marker 33 is used as an indicator indicating a neutral position of the rotational operating portion 27. The hand position markers 33 are provided, for example, one each on the upper case 22a and the lower case 22b, at positions facing the respective protruding portions 27b of the rotational operating portion 27 in the neutral position. By providing the hand position markers 33 on both the top and bottom surfaces of the handle 22, the position of the rotational operating portion 27 can be easily checked even when the handle 22 is rotated. The shape and size of the hand position markers 33 are not particularly limited. The hand position markers 33 are, for example, circular markers having a diameter of 0.5 mm to 5 mm.

The first tube 25 extending from the proximal end of the handle 22 is provided with the locking portion 30 that locks relative movement of the endoscope 10 with respect to the catheter shaft 21. The first tube 25 is a thin tube into which the insertion portion 11 of the endoscope 10 is inserted, and is connected to the catheter shaft 21 within the handle 22 to thereby constitute the first channel 23. The locking portion 30 fastens and locks the insertion portion 11, thereby preventing relative rotation of the insertion portion 11 with respect to the catheter shaft 21. The locking portion 30 also prevents movement of the insertion portion 11 along the longitudinal direction of the catheter shaft 21.

The locking portion 30 is provided at or near the proximal end of the first tube 25. The length of the first tube 25 extending from the proximal end of the handle 22 is, for example, 100 mm or less, and thus the locking portion 30 is located near the proximal end of the handle 22. This allows the locking portion 30 to be easily operated. The physician sets the top of the endoscope 10 to be aligned with the distal end marker 32 and then locks the insertion portion 11 by means of the locking portion 30, thereby maintaining the initial setting. When the locking by the locking portion 30 is insufficient or the insertion portion 11 is rotated by a force exceeding the fastening torque of the locking portion 30, and when it is difficult to determine the top and bottom of the endoscope 10, the top-and-bottom direction can be easily readjusted by means of the distal end marker 32.

The locking portion 30 may be any member that can be attached to the first tube 25 and can lock the insertion portion 11 of the endoscope 10. There are no particular limitations on the structure of the locking portion 30. The locking portion 30 is, for example, a member that has a generally cylindrical shape overall, and includes a passage through which the insertion portion 11 can be inserted, a rubber valve that opens and closes the passage, and an operating portion on its outer circumferential surface. The locking portion 30 is structured so that the rubber valve can be opened and closed by rotating the operating portion. The rubber valve abuts against the outer circumferential surface of the insertion portion 11, thereby locking the relative movement of the insertion portion 11 with respect to the catheter shaft 21.

As in the present embodiment the branch port 31 for irrigation is provided on the first tube 25, it is preferable that the locking portion 30 be provided on the proximal end side of the first tube 25 relative to the branch port 31. By doing so, irrigation of the endoscope 10 can be performed while the locking portion 30 keeps the insertion portion 11 fastened and locked. The locking portion 30 is, for example, disposed adjacent to the proximal end side of the branch port 31.

As described above, the branch port 31 is a branch path for irrigation to supply the cleaning solution to the distal end of the endoscope 10 through the first channel 23. The branch port 31 is attached to the first tube 25. The branch port 31 is provided between the handle 22 and the locking portion 30, to thereby form a cleaning solution supply port in the first channel 23 that is separate from an inlet through which the insertion portion 11 of the endoscope 10 is inserted. That is, by branching the first tube 25, the cleaning solution can be supplied through the first channel 23 with the insertion portion 11 inserted in the first tube 25 and locked by the locking portion 30. The cleaning solution is, for example, physiological saline.

To enable the cleaning solution to flow in the direction of the catheter shaft 21, the branch port 31 is formed so that it gradually extends toward the distal end side of the catheter shaft 21 as it extends from an opening of the port toward a portion connected to the first tube 25. As shown in FIG. 4, the branch port 31 can preferably be configured to be connected to a needleless syringe 51 filled with the cleaning solution. The cleaning solution introduced from the syringe 51 connected to the branch port 31 flows through a gap between the inner circumferential surface of the first channel 23 and the outer circumferential surface of the insertion portion 11 and is supplied to the distal end surface of the insertion portion 11.

The branch port 31 may be provided by attaching, to the first tube 25, a member in which are formed a first path into which the insertion portion 11 is inserted and a second path branching from the first path. The first path constitutes a portion of the first tube 25, and the second path serves as the branch port 31. The member constituting the branch port 31 may be fixed to the handle 22 or may be integrated with the locking portion 30.

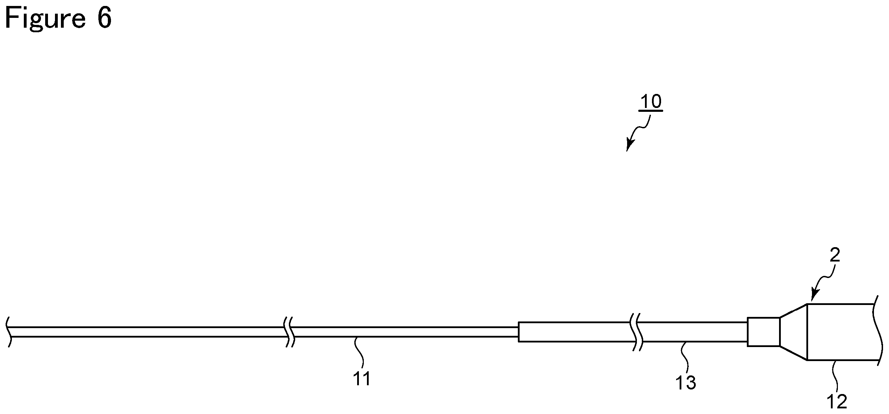

The configuration of the endoscope 10 will be described below in further detail with reference to FIGs. 6 to 8. FIG. 6 is a plan view of the endoscope 10. FIG. 7 is a cross-sectional view taken along the longitudinal direction of a portion of the endoscope 10 on which the protective tube 13 is mounted, and FIG. 8 is a cross-sectional view taken along line AA in FIG. 7.

As shown in FIGs. 6 to 8, the endoscope 10 is provided with the insertion portion 11 and the base portion 2 including the connector portion 12 and the protective tube 13. The endoscope 10 has a structure wherein the protective tube 13 is provided from the connector portion 12 to the proximal end portion of the insertion portion 11. The connector portion 12 is connected to a cable to a console and is also connected to a cable extending from the proximal end portion of the insertion portion 11. The cable includes an electric wire 17 for transmitting electrical signals from the imaging unit 16 and an optical fiber 18 for transmitting illumination light.

The insertion portion 11 has an outer tube 15 that contains the electric wire 17 and the optical fiber 18, and is flexible and bendable. The outer tube 15 is made of, for example, the same material as the catheter shaft 21. Although in the insertion portion 11 which is inserted in the endoscopic treatment instrument 20 the electric wire 17 and the optical fiber 18 are contained in the outer tube 15, portions of the electric wire 17 and the optical fiber 18 extend from the proximal end of the outer tube 15 and are connected to the connector portion 12. The protective tube 13 covers and protects the cable extending from the outer tube 15 so that it is not exposed.

The physician, who is the user, may grasp the connector portion 12 and the protective tube 13 when inserting and setting the endoscope 10 into the endoscopic treatment instrument 20, as well as when using the endoscope system 1. For example, the physician may grasp the protective tube 13 and attempt to rotate it, in order to change the orientation of the endoscope 10. At this time, if the locking by the locking portion 30 is insufficient or the endoscope 10 is rotated by a force exceeding the fastening torque of the locking portion 30, it is conceivable that the insertion section 11 rotates within the first channel 23, and it becomes impossible to determine the top and bottom of the endoscope 10.

To solve the above problem, the endoscope 10 is configured such that the protective tube 13 is not fixed to the insertion portion 11 or the cable, but is instead slidable with respect to the insertion portion 11. In general, the protective tube is fixed to the insertion portion by means of an adhesive or the like. In this case, when a force that causes the protective tube to rotate is applied, the insertion portion 11 rotates in conjunction with the protective tube. In contrast, for the endoscope 10, the protective tube 13 idles, and the insertion portion 11 does not rotate in conjunction with the protective tube 13. This effectively reduces the occurrence of the above problem.

The distal end of the protective tube 13 extends to a position at which it overlaps the insertion portion 11 to avoid exposition of the cable. The protective tube 13 may be longer than the insertion portion 11. The insertion portion 11 has a length that exceeds that of the endoscopic treatment instrument 20. The material of the protective tube 13 is, for example, an elastomer, but may be any material that can protect the cable and has good sliding properties. The protective tube 13 may be adhesively fixed to the distal end section of the connector portion 12.

It is further preferable that the base portion 2 of the endoscope 10 has an inner tube 14 which is a second protective tube. The inner tube 14 contains the proximal end portion of the insertion portion 11, the electric wire 17, and the optical fiber 18, and is disposed within the protective tube 13. The protective tube 13 is not fixed to the inner tube 14 and is configured to be slidable with respect to the inner tube 14. That is, the protective tube 13 is configured to be slidable with respect to the insertion portion 11 via the inner tube 14. This effectively prevents the insertion portion 11 from rotating in conjunction with the protective tube 13.

The inner tube 14 is provided from the distal end of the connector portion 12 to the proximal end portion of the insertion portion 11 to cover the entire length of the cable extending from the outer tube 15. The cable is protected with the two tubes, and thus, it can be protected more reliably. It is preferable that, like the protective tube 13, the inner tube 14 is made of elastomer and exhibits excellent sliding properties with respect to the protective tube 13. The length of the inner tube 14 may be substantially the same as that of the protective tube 13.

It is preferable that the gap between the insertion portion 11 and the inner tube 14 be sealed with an adhesive 19. The adhesive 19 is provided so as to fill the gap, thereby preventing water or the like from entering the interiors of the insertion portion 11 and the inner tube 14. A resin that can firmly bond the insertion portion 11 and the inner tube 14 and has excellent water resistance can be used as the adhesive 19. The adhesive 19 is filled, for example, from the distal end of the inner tube 14 beyond the range in which the inner tube 14 overlaps the insertion portion 11. Meanwhile, because the two protective tubes are not adhesively fixed, the effect of preventing rotation of the insertion portion 11 can be ensured.

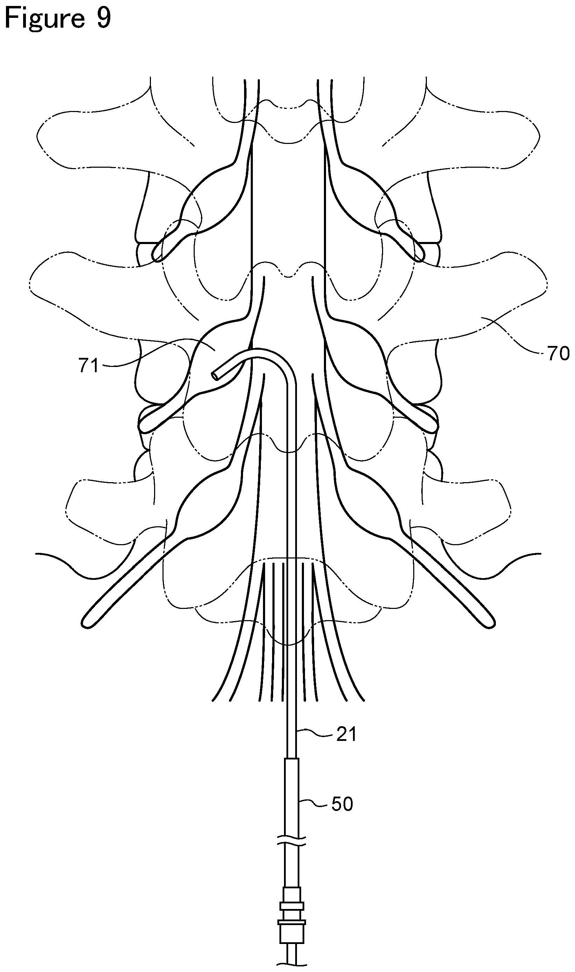

The mechanism of the endoscope system 1 having the above configuration will be further described below with reference to FIG. 9. FIG. 9 is a schematic diagram showing the endoscope system 1 being used for the Racz catheter method.

As shown in FIG. 9, the endoscope system 1 is used by inserting the catheter shaft 21 of the endoscopic treatment instrument 20 into the body. The insertion portion 11 of the endoscope 10 is inserted into the first channel 23 of the catheter shaft 21 and is locked by the locking portion 30 to prevent rotation of the insertion portion 11 with respect to the catheter shaft 21. In the Racz catheter method, a physician inserts the catheter shaft 21 on which a sheath 50 is mounted into the body from the sacrum and advances the catheter shaft 21 extending from the sheath 50 along a lumbar spine 70 to a location near a painful site, while viewing images from the endoscope 10.

The Racz catheter method is attracting attention as a treatment for chronic low back pain caused by conditions such as spinal canal stenosis and herniated discs. It involves inserting the catheter shaft 21 near a painful site to remove adhesions around a nerve root 71. One of the causes of lower back pain is inflammation of the nerve root 71. Fine adhesions exist around the inflamed nerve root 71. Removing these adhesions from around the nerve root 71 can reduce the inflammation of the nerve root 71, thereby alleviating pain.

The adhesions around the nerve root 71 can be peeled off and removed by, for example, supplying a medical solution through the second channel 24 of the catheter shaft 21. It is also possible to remove the adhesions using an instrument, such as forceps or a brush, inserted through the second channel 24. In either case, the physician performs a predetermined treatment while viewing images from the endoscope 10. For example, the physician grasps the handle 22 with the left hand and manipulates the catheter shaft 21 while operating the instrument, such as forceps or a brush, with the right hand. Furthermore, as shown in FIG. 9, the physician can bend the distal end of the catheter shaft 21 by rotating the rotational operating portion 27.

The endoscope system 1 allows the physician to check the top-and-bottom direction of the endoscope 10 using the distal end marker 32 when inserting the insertion portion 11 of the endoscope 10 into the first channel 23. The physician aligns the top of the endoscope 10 with the position of the distal end marker 32 and inserts the insertion portion 11 until the distal end of the insertion portion 11 is substantially aligned with the distal end of the catheter shaft 21. The physician then operates the locking portion 30 to thereby lock the insertion portion 11.

Although, according to the endoscope system 1, the locking portion 30 can lock the endoscope 10 with the top-and-bottom direction of the endoscope 10 specified, when the locking by the locking portion 30 is insufficient, for example, the insertion portion 11 may rotate within the first channel 23, and it becomes impossible to determine the top and bottom of the endoscope 10. Similarly, when the insertion portion 11 or the protective tube 13 is rotated with a force exceeding the fastening torque of the locking portion 30, the same problem may occur.

However, in such cases the top-and-bottom direction of the endoscope 10 inserted in the first channel 23 can be easily checked by means of the distal end marker 32. When the top and bottom of the endoscope 10 cannot be easily determined, the endoscope 10 can be moved a few millimeters toward the proximal end side to check the distal end marker 32, and thus it is not necessary to pull the insertion portion 11 out of the first channel 23 to confirm top and bottom, and the top-and-bottom direction can be easily readjusted.

Furthermore, in the endoscope system 1, the protective tube 13 of the endoscope 10 is configured to be slidable with respect to the insertion portion 11, and thus the insertion portion 11 does not rotate in conjunction with the protective tube 13. The physician may perform an operation such as pinching and twisting the protective tube 13 to change the orientation of the endoscope 10. Even when such an operation is performed, it is possible to effectively prevent rotation of the insertion portion 11 and maintain the initial setting.

The endoscope system 1 makes it possible to supply, from the branch port 31, the cleaning solution to the distal end of the endoscope 10 through the first channel 23 when the distal end of the endoscope 10 becomes contaminated due to adhesion of bodily fluids or the like. As a result, the cleaning solution can be supplied to the distal end of the endoscope 10 more reliably and efficiently. In addition, the branch port 31 is provided on the distal end side of the processing instrument relative to the locking portion 30, and thus irrigation can be easily performed while the endoscope 10 remains locked.

As described above, the endoscope system 1 having the above configuration is simple to operate and easy to use and can greatly reduce the burden on medical professionals using the endoscope system 1.

The above embodiments can be appropriately modified in design within a range that does not impair the object of the present invention. For example, although in the above embodiments the endoscopic treatment instrument 20 provided with the locking portion 30, the branch port 31, and the distal end marker 32 has been described as an example, it is also possible to selectively adopt one or more configuration selected from among these. It is also possible to use a conventionally known endoscope instead of the endoscope 10. However, as described above, the endoscope system 1 having the above configuration provides superior functionality.

Although, in the above embodiments, the optical fiber 18 is incorporated within the insertion portion 11 of the endoscope 10, a light source, such as an LED, may also be disposed on the distal end surface of the insertion portion 11. In addition, although the endoscope provided with an imaging sensor, such as a CMOS image sensor, has been described as an example, it is possible to use an image fiber instead of the imaging device. Although, in the above embodiments, the imaging device (CMOS image sensor) has been used as the image acquisition means, and the optical fiber has been used as the illumination mean, the following configurations are also possible:
Example 1... image acquisition means: image fiber, illumination means: optical fiber;
Example 2... image acquisition means: CMOS image sensor, illumination means: LED; and
Example 3... image acquisition means: image fiber, illumination means: LED.

### REFERENCE SIGNS LIST

1 endoscope system, 2 base portion, 10 endoscope, 11 insertion portion, 12 connector portion, 13 protective tube, 14 inner tube, 15 outer tube, 16 imaging unit, 17 electric wire, 18 optical fiber, 19 adhesive, 20 endoscopic treatment instrument, 21 catheter shaft, 22 handle, 22a upper case, 22b lower case, 23 first channel, 24 second channel, 25 first tube, 26 second tube, 27 rotational operating portion, 27a, 27b protrusions, 28 wire, 29 connector, 30 locking portion, 31 branch port, 32 distal end marker, 33 handle marker, 50 sheath, 51 syringe, 70 lumbar spine, 71 nerve root.

## Claims

1. An endoscopic treatment instrument comprising:
a catheter shaft that has a first channel into which an endoscope is inserted and a second channel used for a predetermined treatment, and
a handle that is attached to a proximal end portion of the catheter shaft, wherein
at a distal end portion of the catheter shaft, a distal end marker for checking the top-and-bottom direction of the endoscope is provided on a portion of an inner circumferential surface of the first channel.

2. An endoscopic treatment instrument comprising:
a catheter shaft that has a first channel into which an endoscope is inserted and a second channel used for a predetermined treatment,
a handle that is attached to a proximal end portion of the catheter shaft, and
a first tube that constitutes the first channel and extends from a proximal end side of the handle, wherein
the first tube is provided with a locking portion that locks relative movement of the endoscope with respect to the catheter shaft.

3. An endoscopic treatment instrument comprising:
a catheter shaft that has a first channel into which an endoscope is inserted and a second channel used for a predetermined treatment,
a handle that is attached to a proximal end portion of the catheter shaft, and
a first tube that constitutes the first channel and extends from a proximal end side of the handle, wherein
the first tube is provided with a branch port for irrigation to supply a cleaning solution to a distal end of the endoscope through the first channel of the catheter shaft.

4. The endoscopic treatment instrument according to any one of claims 1 to 3, wherein the handle is provided with a rotational operating portion for bending the distal end portion of the catheter shaft.

5. The endoscopic treatment instrument according to any one of claims 1 to 4, wherein the handle is provided with a hand position marker.

6. The endoscopic treatment instrument according to any one of claims 1 to 4, wherein
the handle has a maximum thickness of 20 mm to 30 mm, and
the center of the rotational operating portion is disposed within a range of 70 mm to 90 mm from a distal end of the handle and a range of 80 mm to 100 mm from a proximal end of the handle.

7. An endoscope system comprising:
the endoscopic treatment instrument according to any one of claims 1 to 6, and
the endoscope inserted into the first channel.
